# EUROPÄISCHE PATENTSCHRIFT

(11) **EP 2 886 965 B1**
(45) Veröffentlichungstag und Bekanntmachung des Hinweises auf die Patenterteilung: **12.04.2017**
(21) Anmeldenummer: 14197118.4
(22) Anmeldetag: 10.12.2014
(51) Int. Cl.: F24D 17/00, A61L 2/04, C02F 1/02

(54) **WARMWASSERGERÄT UND VERFAHREN ZUM BETREIBEN EINES WARMWASSERGERÄTS**
WARM WATER DEVICE AND METHOD FOR OPERATING A WARM WATER DEVICE
CHAUFFE-EAU ET PROCÉDÉ DE FONCTIONNEMENT D'UN CHAUFFE-EAU

(30) Priorität: 16.12.2013 DE 102013020635
(43) Veröffentlichungstag der Anmeldung: 24.06.2015
(73) Patentinhaber: Stiebel Eltron GmbH & Co. KG, 37603 Holzminden (DE)
(72) Erfinder: Schäfer, Daniel, 32676 Lügde (DE); Grobe, Michael, 37671 Höxter (DE)

(56) Entgegenhaltungen:
- EP-A1- 1 342 957
- EP-A1- 1 693 344
- EP-A1- 1 767 881
- WO-A1-89/03807
- AT-B- 390 943
- DE-A1- 3 838 046
- DE-A1-102009 030 534

## Beschreibung

Die vorliegende Erfindung betrifft ein Warmwassergerät sowie ein Verfahren zum Betreiben eines Warmwassergerätes.

Bei Warmwassergeräten wie beispielsweise bei Durchlauferhitzern, Speichergeräten oder Wärmepumpen, welche längere Warmwasserleitungen versorgen müssen, kann es erforderlich werden, die Warmwasserleitung in regelmäßigen Abständen zu desinfizieren.

DE 38 38 046 beschreibt ein Verfahren zur thermischen Desinfizierung einer zentralen Warmwasserversorgungsanlage. In wählbaren Zeitabständen außerhalb von vorgegebenen Verbrauchszeiten wird das Wasser zur Desinfizierung auf mindestens 60°C erhitzt. Während der Desinfizierung erfolgt eine optische und akustische Warnung zur Vermeidung einer Verbrühung.

EP 1 693 344 A1 beschreibt eine Vorrichtung zur thermischen Desinfektion von Trinkwasserversorgungsanlagen. In einer Zirkulationsleitung ist eine Umwälzpumpe und ein Durchlauferhitzer vorgesehen. Der Durchlauferhitzer wird in vorgegebenen Zeitintervallen zur Desinfektion des Wassers in den Wasserleitungen aktiviert.

DE 10 2009 030534 A1 zeigt ein System für einen automatisierten Betrieb einer elektrisch steuerbaren Wasserversorgungsanlage. Das System kann eine Desinfektionstemperatur besser und zuverlässig kontrollierbar erzeugen.

Es ist eine Aufgabe der vorliegenden Erfindung, ein Warmwassergerät sowie ein Verfahren zum Betreiben eines Warmwassergerätes vorzusehen, welche eine verbesserte und sichere Desinfizierung von Wasserleitungen ermöglichen, die mit dem Gerät verbunden sind.

Diese Aufgabe wird durch ein Warmwassergerät nach Anspruch 1 sowie durch ein Verfahren zum Betreiben eines Warmwassergerätes nach Anspruch 4 gelöst.

Somit wird ein Warmwassergerät, insbesondere ein Durchlauferhitzer, mit einem Gehäuse, einer Gehäusekappe, einem Kanal zum Führen von Wasser und einer Heizeinheit zum Erwärmen des sich in dem Kanal befindlichen Wassers vorgesehen. Das Warmwassergerät weist ferner eine Steuereinheit zum Steuern der Funktion der Heizeinheit bzw. des Warmwassergerätes auf. Die Steuereinheit ist dazu ausgestaltet, das Warmwassergerät in einer ersten Betriebsart (Normalbetriebsart) und zumindest in einer zweiten Betriebsart (Desinfektionsbetriebsart) zu betreiben. In der zweiten Betriebsart wird der Betrieb der Heizeinheit derart gesteuert, dass das in dem Kanal befindliche Wasser auf ≥ 70°C erwärmt wird. Warmwassergerät weist einen Sensor auf zum Erfassen, ob die Kappe geöffnet oder geschlossen ist. Die Steuereinheit ist ferner dazu ausgestaltet, die zweite Betriebsart nur dann zu aktivieren, wenn die Kappe geöffnet ist.

Gemäß einem Aspekt der vorliegenden Erfindung ist die Steuereinheit ferner dazu ausgestaltet, die Desinfektionsbetriebsart nur dann zu aktivieren, wenn seit dem letzten Zapfvorgang ein erstes Zeitintervall vergangen ist.

Gemäß einem weiteren Aspekt der vorliegenden Erfindung ist die Steuereinheit dazu ausgestaltet, die Desinfektionsbetriebsart maximal für ein zweites Zeitintervall zu aktivieren.

Die Erfindung betrifft ebenfalls ein Verfahren zum Steuern eines Warmwassergerätes. Das Warmwassergerät weist einen Wasserkanal, eine Heizeinheit und eine Gerätekappe auf. Eine Desinfektionsbetriebsart wird aktiviert und das sich in dem Kanal befindliche Wasser wird durch die Heizeinheit auf eine Temperatur von ≥ 70°C erwärmt. Gemäß der Erfindung wird zunächst erfasst, ob die Gerätekappe geöffnet ist. Die Desinfektionsbetriebsart wird nur dann aktiviert, wenn die Kappe geöffnet ist.

Die Erfindung betrifft den Gedanken, ein Warmwassergerät mit einer Heizeinheit zum Erwärmen von Wasser vorzusehen, wobei das Warmwassergerät mindestens zwei Betriebsarten, nämlich eine Normalbetriebsart und eine Desinfektionsbetriebsart, aufweist. In einer Desinfektionsbetriebsart wird das Wasser durch die Heizeinheit auf eine Temperatur von > 70°C erwärmt. Die Desinfektionsbetriebsart kann erfindungsgemäß geöffnet nur aktiviert werden, wenn eine Gerätekappe geöffnet worden ist und hierdurch ein Kundendienstmodus aktiviert worden ist. Der Kundendienstmodus könnte alternativ, aber nicht erfindungsgemäß auch durch die Eingabe einer anderen Zugangsberechtigung, beispielsweise durch Eingabe eine PIN-Codes aktiviert werden. Ferner kann die Desinfektionsbetriebsart optional nach einer vorgegebenen Zeit nach der letzten Zapfung und optional nur für ein fest vorgegebenes maximales Zeitintervall durchgeführt werden.

Weitere Ausgestaltungen der Erfindung sind Gegenstand der Unteransprüche. Vorteile und Ausführungsbeispiel der Erfindung werden nachstehend unter Bezugnahme auf die Zeichnung näher erläutert.
- Fig. 1: zeigt ein schematisches Blockschaltbild eines Warmwassergerätes gemäß einem ersten Ausführungsbeispiel, und
- Fig. 2: zeigt ein Flussdiagramm eines Verfahrens zum Steuern eines Warmwassergerätes gemäß einem zweiten Ausführungsbeispiel.

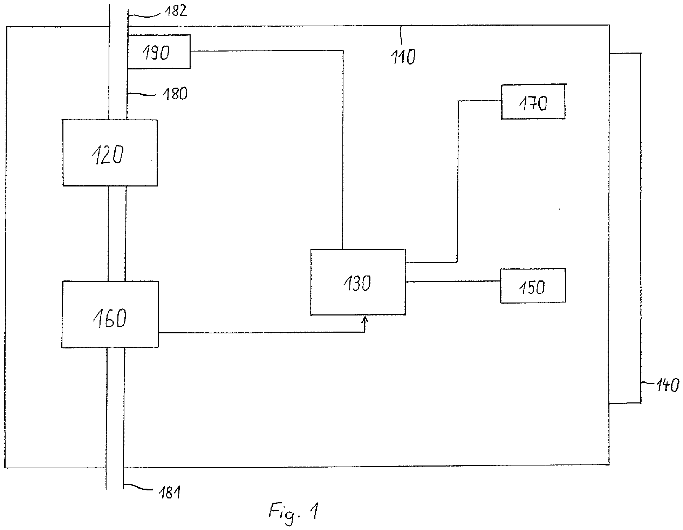

Fig. 1 zeigt ein schematisches Blockschaltbild eines Heißwassergerätes gemäß einem ersten Ausführungsbeispiel.

Das Warmwassergerät 100 weist ein Gehäuse 110, eine Kappe 140, eine Heizeinheit 120 und eine Steuereinheit 130 auf. Optional kann ein Durchflusssensor 160 sowie eine Bedieneinheit 150 vorgesehen sein. Die Steuereinheit 130 ist dazu ausgestaltet, den Betrieb des Warmwassergerätes 100 zumindest in einer ersten Betriebsart (Normalbetriebsart) oder in einer zweiten Betriebsart (Desinfektionsbetriebsart) zu steuern.

In einer Normalbetriebsart wird das Wasser in einem Kanal 180 durch die Heizeinheit 120 entsprechend der angeforderten Wassermenge erwärmt. Die Erwärmung des Wassers kann beispielsweise durch einen Temperatursensor 190 überwacht werden. Ferner kann in der ersten Betriebsart eine maximale Wassertemperatur von beispielsweise 60°C vorgesehen sein. Dies kann durch den Temperatursensor 190 und die Steuereinheit 130 überwacht werden.

In einer zweiten Betriebsart, nämlich der Desinfektionsbetriebsart, steuert die Steuereinheit 130 die Heizeinheit 120 so, dass das Wasser innerhalb des Kanals 180 auf eine Temperatur > 70°C, beispielsweise 75°C, erwärmt wird. Anschließend kann das derart erwärmte Wasser durch die Wasserleitungen fließen und somit zu einer Desinfektion der Wasserleitungen verwendet werden.

Die Steuereinheit 130 ist ferner dazu ausgestaltet, die zweite Betriebsart, nämlich die Desinfektionsbetriebsart, lediglich dann zu aktivieren, wenn mittels des Sensors 170 erfasst wird, dass die Gerätekappe 140 geöffnet bzw. entfernt wird und/oder wenn optional eine Kundendienstbetriebsart mittels der Bedieneinheit 150 durch den Kundendienstmitarbeiter ausgewählt wird. Ferner kann die Steuereinheit 130 mittels des Durchflusssensors 170 den Durchfluss des sich in dem Kanal 180 befindlichen Wassers erfassen. Die Steuereinheit 130 kann ferner erfassen, wann der letzte Zapfvorgang erfolgt ist. Dies kann sie mittels der Ausgangswerte des Durchflusssensors 160 erfassen.

Um die Betriebssicherheit des Warmwassergerätes in der Desinfektionsbetriebsart (zweite Betriebsart) zu verbessern, wird die zweite Betriebsart durch die Steuereinheit 130 optional nur dann aktiviert, wenn seit dem letzten Zapfvorgang ein vorgegebenes Zeitintervall vergangen ist. Wenn das vorgegebene Zeitintervall abgelaufen ist, kann die Steuereinheit 130 die zweite Betriebsart aktivieren, so dass das sich in dem Kanal befindliche Wasser mittels der Heizeinheit 120 auf über 70°C erwärmt wird. Die Steuereinheit 130 kann ferner die Zeitdauer der zweiten Betriebsart überwachen und kann optional den Betrieb der zweiten Betriebsart zeitlich begrenzen. Dies ist ebenfalls vorteilhaft hinsichtlich der Betriebssicherheit des Heißwassergerätes.

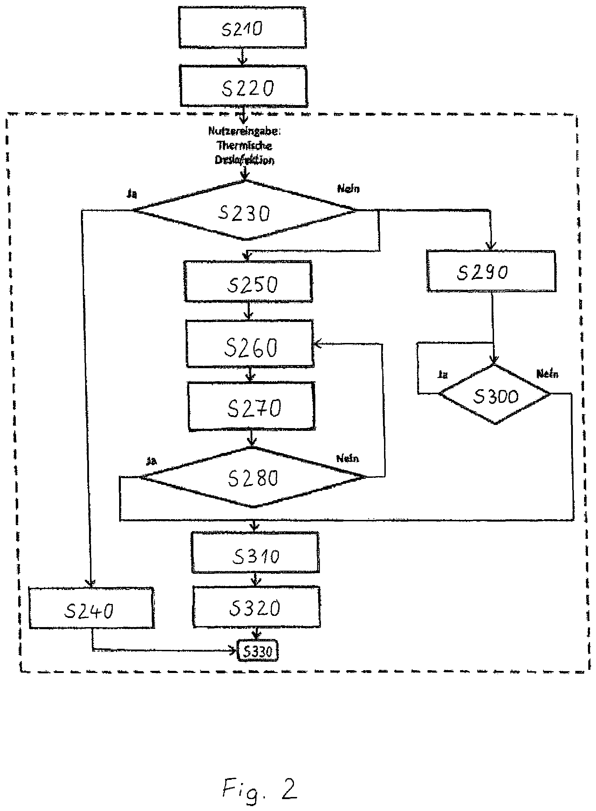

Fig. 2 zeigt ein Flussdiagramm eines Verfahrens zum Steuern eines Warmwassergerätes gemäß der Erfindung. Das Warmwassergerät gemäß dem zweiten Ausführungsbeispiel kann dem Warmwassergerät gemäß dem ersten Ausführungsbeispiel entsprechen.

In Schritt S210 wird die Gerätekappe 140 abgenommen und in Schritt S220 wird eine Kundendienstbetriebsart durch Betätigung der Bedieneinheit 150 aktiviert. Durch eine entsprechende weitere Betätigung der Bedieneinheit 150 kann dann eine Desinfektionsbetriebsart aktiviert werden. In Schritt S230 wird durch die Steuereinheit 130 mittels des Durchflusssensors 160 überprüft, ob ein Durchfluss innerhalb eines ersten Zeitintervalls (z. B. in den letzten zwei Minuten) erfolgt ist. Wenn ein Durchfluss innerhalb des ersten Zeitintervalls erfolgt ist, dann wird in Schritt S240 die Aktivierung der Desinfektionsbetriebsart blockiert und ein entsprechender Hinweis kann ausgegeben werden. Anschließend endet das Verfahren in Schritt S330.

Wenn jedoch in Schritt S230 erfasst wird, dass kein Durchfluss durch den Kanal 180 innerhalb des ersten Zeitintervalls erfolgt ist, dann wird in Schritt S290 ein Timer (der z. B. in der Steuereinheit 130 implementiert ist) gestartet und die Solltemperatur der Heizeinheit 120 wird in Schritt S250 durch die Steuereinheit 130 auf über 70°C, beispielsweise 75°C, eingestellt. In Schritt S260 kann ein akustischer und/oder optischer Warnhinweis ausgegeben werden, um ein Verbrühen während der Desinfektionsbetriebsart zu vermeiden. In Schritt S260 und S270 kann eine entsprechend akustische und/oder optische Warnung auf einer Fernbedienung des Gerätes und in oder an dem Gehäuse 110 des Gerätes dargestellt werden.

Wenn in Schritt S280 erfasst wird, dass die letzte Zapfung nicht länger als das erste Zeitintervall her ist, dann wird der Fluss zu Schritt S260 geleitet. Wenn jedoch in Schritt S280 erfasst wird, dass die letzte Zapfpause länger als das erste Zeitintervall her ist, dann schreitet der Fluss zu Schritt S310 sowie zu Schritt S300 weiter. In Schritt S300 wird bestimmt, wie der Stand des Timers ist (d. h. seit wann ist die Desinfektionsbetriebsart aktiviert) bzw. ob ein maximaler Wert (wie beispielsweise 15 Minuten) des Timers überschritten worden ist. Wenn dies nicht der Fall ist, dann schreitet der Fluss von Schritt S300 zu Schritt S310 weiter. In Schritt S310 werden die Nutzereinstellungen, d. h. die Normalbetriebsart, wieder hergestellt. In Schritt S320 kann auf der Fernbedienung und/oder auf dem Gerät angezeigt werden, dass die Desinfektion beendet worden ist.

## Patentansprüche

1. Warmwassergerät, insbesondere Durchlauferhitzer, mit
einem Gehäuse (110),
einem Kanal (180) zum Führen von Wasser,
einer Heizeinheit (120) zum Erwärmen des sich in dem Kanal (180) befindlichen Wassers
und
einer Steuereinheit (130) zum Steuern der Funktion der Heizeinheit (120) und zum Betreiben des Warmwassergeräts in einer ersten Betriebsart, welche die normale Betriebsart darstellt, und zumindest in einer zweiten Betriebsart, wobei die zweite Betriebsart eine Desinfektionsbetriebsart darstellt,
wobei die Steuereinheit (130) in der zweiten Betriebsart dazu ausgestaltet ist, den Betrieb der Heizeinheit (120) derart zu steuern, dass das sich in dem Kanal (180) befindliche Wasser auf ≥ 70°C erwärmt wird, **dadurch gekennzeichnet, dass**
das Warmwassergerät eine Kappe (140) und einen Sensor (170) zum Erfassen, ob die Kappe (140) geöffnet ist oder nicht, aufweist und
wobei die Steuereinheit (130) ferner dazu ausgestaltet ist, die zweite Betriebsart nur dann zu aktivieren, wenn die Kappe (140) geöffnet ist.

2. Warmwassergerät nach Anspruch 1, wobei
die Steuereinheit (130) ferner dazu ausgestaltet ist, die zweite Betriebsart nur dann zu aktivieren, wenn seit einem letzten Zapfvorgang ein vorgegebenes erstes Zeitintervall vergangen ist.

3. Warmwassergerät nach einem oder mehreren der Ansprüche 1 bis 2, wobei
die Steuereinheit (130) dazu ausgestaltet ist, die zweite Betriebsart maximal für ein zweites Zeitintervall zu aktivieren.

4. Verfahren zum Steuern eines Warmwassergerätes, wobei das Warmwassergerät einen Wasserkanal (180), eine Heizeinheit (120) und eine Gerätekappe (140) aufweist, **gekennzeichnet durch** die Verfahrensschritte: Erfassen, ob die Gerätekappe (140) geöffnet ist, und wenn die Kappe (140) geöffnet ist, Aktivieren einer Desinfektionsbetriebsart, bei der das sich in dem Kanal (180) befindliche Wasser auf eine Temperatur von ≥70°C **durch** die Heizeinheit (120) erwärmt wird,

5. Verfahren zum Steuern eines Warmwassergerätes nach Anspruch 4, ferner mit den Schritten:
Erfassen, wann der letzte Zapfvorgang erfolgt ist und
Aktivieren der Desinfektionsbetriebsart, wenn seit dem letzten Zapfvorgang ein erstes Zeitintervall vergangen ist.

6. Verfahren nach einem oder mehreren der Ansprüche 4 bis 5, wobei
die Zeitdauer der Aktivierung der Desinfektionsbetriebsart überwacht wird,
wobei die Desinfektionsbetriebsart beendet wird, wenn die Desinfektionsbetriebsart länger als ein zweites Zeitintervall aktiviert ist.

## Claims

1. Hot water device, in particular flow-through heater, with
one housing (110),
one conduit (180) for routing water,
one heating unit (120) for heating the water contained in the conduit (180) and
one control unit (130) for the control of the function of the heating unit (120) and for the operation of the hot water device in a first operating mode, which represents the normal operating mode, and at a minimum in a second operating mode, where the second operating mode represents a disinfection operating mode,
where the control unit (130) in the second operating mode is designed in order to control the operation of the heating unit (120) in such a way that water contained in the conduit (180) is heated to ≥ 70°C,
**characterised in that**
the hot water device indicates a cap (140) and a sensor (170) for recording whether or not the cap is open (140), and
where the control unit (130) is further designed to activate the second operating mode only if the cap is open (140).

2. Hot water device according to Claim 1, where
the control unit (130) is further designed to activate the second operating mode only if a specified first time period has expired since a last tapping procedure.

3. Hot water device according to one or more of the Claims 1 to 2, where
the control unit (130) is also designed to activate the second operating mode for a second time period maximum.

4. Process for the control of a hot water device, where the hot water device indicates one water conduit (180), one heating unit (120) and one device cap (140), identified by the procedural steps:
Recording whether the device cap is opened (140),
and
if the cap is opened (140), activation of a disinfection operating mode, where the water contained in the conduit (180) is heated to ≥ 70°C by the heating unit (120).

5. Process for the control of a hot water device according to Claim 4, with the further steps:
Recording when the last tapping procedure was implemented and
activation of the disinfection operating mode if an initial time period has expired since the last tapping procedure.

6. Process according to one or more of the Claims 4 to 5, where
the period of the activation of the disinfection operating mode is monitored,
where the disinfection operating mode is ended if the disinfection operating mode is activated longer than a second time period.

## Revendications

1. Dispositif à eau chaude, plus précisément chauffe-eau instantané, comprenant
un châssis (110),
un canal (180) pour l'acheminement de l'eau,
une unité de chauffe (120) destinée à chauffer l'eau se trouvant dans le canal (180)
et
une unité de commande (130) destinée à commander le fonctionnement de l'unité de chauffe (120) et celui du dispositif à eau chaude selon un premier mode de fonctionnement, représentant le mode de fonctionnement normal, et au moins selon un second mode de fonctionnement, le second mode de fonctionnement représentant un mode de désinfection,
l'unité de commande (130) étant, dans le second mode de fonctionnement, conçue afin de commander l'unité de chauffe (120) de manière à ce que l'eau se trouvant dans le canal (180) soit chauffée à ≥ 70°C,
**caractérisé en ce que**
le dispositif à eau chaude présente un couvercle (140) et un capteur (170) destiné à détecter si le couvercle (140) est ouvert ou non, et
**en ce que** l'unité de commande (130) est également conçue afin d'activer le second mode de fonctionnement uniquement si le couvercle (140) est ouvert.

2. Dispositif à eau chaude selon la revendication 1, **caractérisé en ce que**
l'unité de commande (130) est également conçue afin d'activer le second mode de fonctionnement uniquement si, depuis une dernière opération de soutirage, un premier intervalle de temps déterminé s'est écoulé.

3. Dispositif à eau chaude selon l'une ou plusieurs des revendications 1 à 2, **caractérisé en ce que**
l'unité de commande (130) est conçue afin d'activer le second mode de fonctionnement au maximum pendant un second intervalle de temps.

4. Procédé de commande d'un dispositif à eau chaude, le dispositif à eau chaude présentant un canal d'eau (180), une unité de chauffe (120) et un couvercle (140), **caractérisé par** les étapes de procédé :
détecter si le couvercle (140) est ouvert,
et
si le couvercle (140) est ouvert, activer un mode de désinfection pendant lequel l'eau se trouvant dans le canal (180) est chauffée à une température de ≥70°C par l'unité de chauffe (120).

5. Procédé de commande d'un dispositif à eau chaude selon la revendication 4, se poursuivant avec les étapes :
détecter lorsque la dernière opération de soutirage a été réalisée et
activer le mode de désinfection si, depuis la dernière opération de soutirage, un premier intervalle de temps s'est écoulé.

6. Procédé selon l'une ou plusieurs des revendications 4 à 5, **caractérisé en ce que**
le délai d'activation du mode de désinfection est surveillé,
le mode de désinfection prenant fin si le mode de désinfection est activé pendant plus longtemps qu'un second intervalle de temps.
